# EUROPEAN PATENT APPLICATION

(11) **EP 4 806 452 A1**
(43) Date of publication of application: **16.09.2026**
(21) Application number: 25162587.7
(22) Date of filing: 10.03.2025
(51) Int. Cl.: A61K 38/17, A61K 48/00, C12N 15/864, A61P 9/04

(54) **INDUCTORS OF ICER, GPCR65 AND/OR GPCR132 AND THEIR USE IN THE PREVENTION OR TREATMENT OF HEART FAILURE**

(71) Applicant: Universitätsmedizin der Johannes Gutenberg-Universität Mainz, 55131 Mainz (DE)
(72) Inventor: WENZEL, Philip Christian, 55128 Mainz (DE); MOLITOR, Michael, 55131 Mainz (DE); BOPP, Tobias, 65187 Wiesbaden (DE); BOHN, Toszka, 55128 Mainz (DE)
(74) Representative: Patentanwälte Dr. Keller, Schwertfeger Partnerschaft mbB

(57) **Abstract**

The present invention identifies novel targets for the prevention or treatment of heart failure. In more particular, the present invention relates to inductors of G-protein coupled receptor (GPCR)-mediated acidic stress response in cardiac or circulating bone marrow-derived monocytes or macrophages and vehicles for delivering a target mRNA or cDNA modulating the expression of ICER, GPCR65 and/or GPCR132. The present invention also relates to monocyte or macrophage cells transfected with a vehicle comprising an inductor of ICER, GPCR65 and/or GPCR132 gene or protein expression.

## Description

**The present** invention identifies novel targets for the prevention or treatment of heart failure.

In more particular, the present invention relates to inductors of G-protein coupled receptor (GPCR)-mediated acidic stress response in cardiac or circulating bone marrow-derived monocytes or macrophages and vehicles for delivering a target mRNA or cDNA modulating the expression of ICER, GPCR65 and/or GPCR132. The present invention also relates to monocyte or macrophage cells transfected with a vehicle comprising an inductor of ICER, GPCR65 and/or GPCR132 gene or protein expression.

Cardiovascular diseases (CVD) are among the most common causes of mortality and morbidity worldwide^{1,2}. Ischemic heart disease is the most common global cause of death. Cardiovascular risk factors, such as diabetes mellitus, smoking, dyslipidemia or arterial hypertension, are particularly responsible for the development of the disease." These factors lead to a narrowing of the arterial blood vessels due to atherosclerosis, which is caused by cholesterol deposits and monocytes/macrophage-driven vascular inflammation ⁴⁻⁶. An acute myocardial infarction often represents a dramatic endpoint in the course of the disease. Despite considerable improvements in acute therapy, e.g., through treatment with percutaneous coronary intervention (PCI) during cardiac catheterization, maladaptive cardiac remodeling, which causes ischemic heart failure, remains a crucial clinical challenge. This challenge has not yet been satisfactorily resolved, particularly in patients who seek medical attention late after the onset of heart attack symptoms and can therefore receive medical care outside the optimal time for revascularization^{7-10.}

The current treatment options for ischemic heart disease include the control of
cardiovascular risk factors hyperlipoproteinemia¹¹, arterial hypertension¹², diabetes mellitus¹³ and smoking and is thus a primary-preventive approach¹⁴ and in case of manifest CVD a secondary-preventive approach¹⁵. In the setting of ACS (acute coronary syndrome), timely revascularization is warranted, preferably by cardiac catheterization with percutaneous coronary intervention (PCI), particularly in the most severe forms of ACS, the STEMI (ST-segment elevation myocardial infarction)¹⁶. However, the effectiveness decreases rapidly if more than 24 hours have passed since the onset of symptoms, as ischemic tissue has then perished and can no longer be saved. Subsequently, a process called post-infarction or ischemic remodeling sets in, which is characterized by excess fibrosis processes leading to the development of heart failure.

Anti-inflammatory therapies for secondary prevention in high-risk patients after myocardial infarction are available as complementary therapeutic approaches, although they have not progressed beyond the stage of very non-selective immunomodulation with colchicine; they are therefore only recommended as complementary therapy for selected patients¹⁴.

One aim of a therapeutic approach is to prevent heart failure as a consequence of an untimely revascularized or recanalized myocardial infarction. Currently no drug therapy is available that could prevent excessive fibrosis and thus the development of heart failure, and therapeutic approaches include a control of risk factors such as hypertension and diabetes for the development of heart failure in long-term^{17,18}.

It is also known that the occlusion of a coronary vessel with reduced supply of myocardial tissue is followed by the development of tissue hyperacidity due to the production of lactate and metabolic acid products^{19-21.} The development of heart failure, especially after myocardial infarction, is caused by prolonged metabolic stress due to an imbalance in oxygen/nutrient demand and supply, in impaired cellular energy turnover and an excessive immune response. Prolonged hypoxia and increased anaerobic glycolysis lead to an acidic environment in the affected tissue. This acidosis results in a polarization of myeloid cells mainly via second messenger molecule cyclic AMP (cAMP) and GPCRs ^{22,23}. An important signaling pathway is the transcriptionally inducible early repressor of cAMP (inducible cAMP early repressor), abbreviated to ICER.

cAMP enables immune cells to react to different pH values of a tissue and change their phenotype. Malignant tumor cells use this effect to prevent themselves from being recognized and attacked by immune cells. However, the significance of metabolic acid stress on cardiac and vascular immune cells and how it affects their function and role in tissue homeostasis, (mal)adaptation and repair, is still poorly understood²⁴.These repair processes are crucial for survival and maintenance of cardiac and vascular function; however, an excessive immune response can also significantly disrupt the repair process. If the regulatory mechanisms of the healing process are disturbed, this can lead to a defective scar, dysfunction and adverse remodeling of the heart muscle, which in turn leads to the development of heart failure, cardiac arrhythmias and excess mortality^{9, 25-27}.

WO 2004/013285 A2 describes methods of identifying whether a candidate compound is a modulator of an orphan GPCR. Furthermore, a method of prevention or treatment of a cardiovascular disorder is disclosed, comprising contacting a therapeutically effective amount of a ROP41 GPCR modulator. A similar approach is disclosed in JP 2011 162 567 A, which relates to a composition for preventing or treating a disorder using an inverse agonist of a ROP40 GPCR.

Against this background, it is the object of the present invention to provide alternative targets and therapeutically suitable compounds for the prevention and treatment of ischemic and non-ischemic heart failure as a result of metabolic acidic stress.

This object is solved by an inductor of GPCR-mediated acidic stress response in cardiac or circulating bone marrow-derived monocytes or macrophages, wherein the inductor mediates induction of inducible cAMP early repressor (ICER), GPCR65 and/or GPCR132 for use in the prevention or treatment of heart failure (HF).

The present invention characterizes new targets for the prevention or treatment of HF and its decisive risk factors such as arterial hypertension and diabetes mellitus. To achieve cardiac protection, the present invention targets metabolic signaling pathways and the modulatory role of ICER in monocytes and macrophages, especially after acute myocardial infarction and in arterial hypertension. In more particular, the invention targets the transcription factor ICER and the acid-sensitive receptors GPCR65 and GPCR132 in myeloid cells, which decisively influence cellular cAMP levels and thus the polarization of immune cells. In more particular, the invention identifies ICER, GPCR65 and GPCR132 as potential targets to modulate tissue acidosis and aims to restore their function, activity or expression levels in cardiac or circulating bone marrow-derived monocytes or macrophages. These cells are essential in the initial inflammatory response to ischemia, wound healing and reservation of tissue homeostasis, and post-infarction repair processes are critical for survival.

GPCR65 and GPCR132 have in common that they belong to the same pathway and both can be activated by extracellular acidic pH through protonation of histidine residues of the receptors. Following activation, several downstream G-protein-coupled pathways are activated and transcription factors like ICER are induced. The invention is based on the surprising finding that by inducing ICER, GPCR65 or GPCR132 expression or by restoring their function, tissue homeostasis and the supply with oxygen and nutrients to organism in cardiovascular diseases is maintained thereby counteracting the acidification of tissue.

The present invention presents both in vitro and in vivo data showing that ICER, GPCR65 and/or GPCR132 are therapeutically valuable targets in the treatment of HF. Modulation of these proteins can occur both at the level of gene expression, protein expression, and may include post-translational processes, protein modification, RNA stabilization as well as restoring ICER, GPCR65 and/or GPCR132 function by chemical or biological processes. As exemplified herein, the inventors have isolated bone marrow-derived monocytes from donor animals in which the myeloid cells are ICER-competent (LysM^{wt/wt} ICER^{flox/flox}, wildtype controls) and transferred them into recipient animals that specifically lack ICER in lysozyme M-positive myeloid cells (LysM^{Cre/wt} ICER^{flox/flox}). Accordingly, it could be demonstrated that the transfer of ICER-competent monocytes was able to restore the cardiac phenotype to the level of the wildtype controls with an improvement in left ventricular ejection fraction.

Furthermore, in a mouse model of non-reperfused myocardial infarction, the present invention demonstrates an upregulation of GPCR65 and GPCR132. The cellular mechanisms for the perception of metabolic acid stress through ICER, GPCR65 and GPCR132 are crucial to initiate repair processes after HF, in particular after non-reperfused myocardial infarction. As shown herein, the inactivation of ICER, GPCR65 and/or GPCR132 in myeloid cells modulates their phenotype and deteriorates cardiac function in non-reperfused MI and can be rescued by means of ICER-competent BM-derived monocytes. Modulation of GPCR-coupled acidic stress response involving the induction of ICER has the potential to attenuate post-MI cardiac remodeling and contributes to vascular protection, prevents developments of ischemic HF and vascular disfunction, and maintains tissue homeostasis in case of nutrient/oxygen deprivation and acidification in cardiovascular diseases.

The results of the present invention suggest that a wide variety of HF diseases can be treated or prevented, in particular ischemic HF, myocardial infarction (MI), IHF resulting from acute and/or ongoing MI, HF with reduced ejection fraction (HFrEF), or HF with preserved ejection fraction (HFpEF), HF resulting from vascular dysfunction, disturbed tissue homeostasis and/or tissue acidification.

The inductor of ICER, GPCR65 and/or GPCR132 can be any nucleic acid, polypeptide, protein, agent, compound, substance, transcription factor, transcription enhancer or biological system (such as non-viral or viral vehicles) that modulates, regulates, restores and/or maintains ICER, GPCR65 and/or GPCR132 activity or function in myeloid cells, in particular in cardiac or circulating bone marrow-derived monocytes or macrophages. The present invention comprises both non-viral delivery methods and viral delivery methods in order to deliver mRNA or cDNA encoding ICER, GPCR65 and/or GPCR132 to their target monocytes or macrophages. In preferred embodiments, the inductor of the invention is an expression construct, a proximity inducing drug, CREM, CD68, CSF1R or HIF-1α promoter, RNA stabilizer, transcription factor, or an enhancer of ICER, GPCR65 or GPCR132 gene or protein expression or agonist of GPCR65 or GPCR132. The expression construct can carry mRNA-coding sequences and can be both linear mRNA or plasmid-based DNA. In alternative embodiments, specific promoters can be used to trigger or enhance gene expression of ICER, GPCR65 or GPCR132 genes. For example, cell-type specific promoters can be used such as CSF1R, CD68, or TIE2.

Enhancers as used in the present invention can boost transcription or transcription efficiency of ICER, GPCR65 or GPCR132 transcription and gene expression. Also, engineered expression constructs can be utilized in order to internally or externally express ICER, GPCR65 or GPCR132 gene expression. Such constructs can be regulated by specific stress pathways such as HIF-1α (hypoxia), NF-κB (inflammation), AP-1 (acidic stress).

In a first aspect of the present invention, the enhancer for ICER can be anyone of cAMP response element (CRE) enhancers, NK-κb enhancers, HIF-1α enhancers or CREB-dependent calcium-responsive element enhancers.

In another aspect of the present invention, the enhancer for GPCR65 is anyone of HIF-1α enhancers, hypoxia-inducible elements (HRE) enhancers, NF-κB and AP-1 enhancers, or PU.1 and IRF8-associated enhancers.

In a further aspect of the present invention, the enhancer for GPCR132 is anyone of PPARγ-responsive enhancers, HIF-1α enhancers, hypoxia-inducible elements (HRE) enhancers, or NF-κB and STAT3 enhancers.

In a preferred embodiment, the inductor is a mRNA or cDNA encoding ICER, GPCR65 or GPCR132.

Preferred delivery methods of delivering mRNA to target monocytes or macrophages, i.e., cardiac or circulating bone marrow-derived monocytes or macrophages, include, but are not limited to non-viral delivery methods such as lipid nanoparticles (LNPs), polymeric nanoparticles, cationic peptides, exosomes or extracellular vesicles (EVs), functionalized gold nanoparticles, electroporation or hydrogel-based delivery.

In a preferred embodiment, the inductors of the invention or their targets ICER, GPCR65 or GPCR132 are inducible by an acidic pH environment. For example, exosome-based delivery can comprise monocyte or macrophage-derived exosomes that carry ICER, GPCR65 or GPCR132 mRNA, wherein the exosome is responsive to acidic conditions. Such pH-sensitive exosomes could be modulated under acidic stress following HF. The therapeutic progress can be observed by restoring ICER, GPCR65 or GPCR132 activity in affected myeloid cells.

In alternative embodiments, viral methods can be used for low-term expression of ICER, GPCR65 or GPCR132. Most preferably, cDNAs are delivered to cardiac or circulating bone marrow-derived monocytes or macrophages by means of a viral delivery vehicle, such as adeno-associated viral vector (AAV), adenoviral vector, helper-dependent adenoviral vector (HdAd), lentiviral vector or Sendai virus vector. These viral vectors can allow for stable gene expression in monocytes or macrophages and bear a low risk of genomic integration. AAV delivery systems are efficient and provide long-term gene expression without genomic integration. As such, an AAV is equipped with an inducible promoter that is acidic stress-sensitive, expression of ICER, GPCR65 or GPCR132 can be activated under ischemic conditions. In alternative embodiments, Sendai virus (SeV) can be used for treatment of acute ischemic stress by delivering mRNA for transient gene expression without genomic integration.

For a transient response, LNP-mRNA or exosome-mRNA encoding ICER, GPCR65 or GPCR132 are preferred. For sustained expression, AAV-cDNA encoding ICER, GPCR65 or GPCR132 is preferred. For monocyte reprogramming in ex vivo, a lentiviral delivery system may be preferable.

Enhancers of the invention can also comprise clusters of enhancers that promote ICER, GPCR65 or GPCR132 expression in cardiac or circulating bone marrow-derived monocytes or macrophages. Such enhancers include, but are not limited to AP-1 and PU.1-regulated enhancers. In alternative embodiments, artificial enhancers can be used such as CMV enhancer fused to a specific promoter. Inducible enhancers can be activated by stress signals, such as hypoxia, inflammation or acidic stress, and may comprise, for example, HIF-1α-responsive enhancers.

In some embodiments, NF-κB-responsive enhancers can be used which are activated during inflammation and ischemia, and as such can increase gene expression in monocytes or macrophages under stress. In alternative embodiments, AP-1 and PU.1 enhancers can be used to mediate monocyte-, macrophage-specific expression of ICER, GPCR65 and GPCR132. IRF8 enhancer can be used in order to modulate monocyte to macrophage differentiation and reduced ICER expression in immune cells. Alternative enhancers are tissue-specific and include, but are not limited to TIE2 enhancer, CD68 enhancer or CSF1 R enhancer.

In some embodiments, GPCR-mediated enhancers are preferred, in particular enhancers that cannot be activated by acidic stress or low pH. Such enhancers include, but are not limited to HIF-1α-responsive enhancers, GPR4-responsive enhancers or NFAT and CREB enhancers. They can be activated by GPCR-signaling in response to low pH and ischemic stress and as such are useful for macrophage polarization in acidic cardiac tissue. Alternative specific embodiments can also use Tet-on/Tet-off enhancer systems.

The present invention also relates to a vehicle for delivering a target mRNA or cDNA to cardiac or circulating bone marrow-derived monocytes or macrophages, wherein said target mRNA or cDNA encodes an inductor of GPCR-mediated acidic stress response in cardiac or circulating bone marrow-derived monocytes or macrophages, wherein said inductor modulates expression of inducible cAMP early repressor (ICER), GPCR65 and/or GPCR132 in said cardiac or circulating bone marrow-derived monocytes or macrophages.

In one aspect of the present invention, the vehicle is suitable for use in the prevention or treatment of HF. The inductor included in such a vehicle can be anyone as described herein. The vehicle may also include multiple copies of an inducer according to the present invention. In an alternative embodiment, the vehicle may include genes encoding different inducers of ICER, GPCR65 and/or GPCR132 expression.

In other aspects of the present invention, combinations of a single or different expression systems can be utilized in a method for treating or preventing HF.

In a preferred embodiment, the vehicle is a non-viral delivery vehicle selected from the group consisting of lipid nanoparticles (LNPs), polymeric nanoparticles, cationic peptides, monocyte or macrophage-derived exosomes or extracellular vesicles (EVs), functionalized gold nanoparticles.

In an alternative embodiment, the vehicle is a viral delivery vehicle selected from the group consisting of adeno-associated viral vector (AAV), adenoviral vector, helper-dependent adenoviral vector (HdAd), lentiviral vector or Sendai virus vector.

In a further preferred embodiment, the vehicle allows prevention or treatment of HF, such as IHF, MI, IHF resulting from acute and/or ongoing MI, HFrEF, or HFpEF, HF resulting from vascular dysfunction, disturbed tissue homeostasis and tissue acidification. Preferably, such a vehicle is part of a pharmaceutical composition that is administered to a subject to be treated.

The invention also relates to a monocyte or macrophage cell, comprising a gene encoding ICER, GPCR65 or GPCR132, wherein said cell is transfected with a vehicle comprising an inductor of ICER, GPCR65 or GPCR132 gene or protein expression as defined herein. Such cellular systems can be adapted to be suitable for an in vivo treatment of patients in need thereof. For example, monocyte or macrophage cells can be used in a cell therapy approach in which viral cells that are modified to express ICER, GPCR65 or GCPR132 are injected, grafted or implanted into a patient in order to modulate acidic stress response involving the induction of ICER, GPCR65 and/or GPCR132.

While an inducer of the present invention may be administered directly to a patient, it is preferable to administer the inducer, vehicle, or the modified monocyte or macrophage cells as a pharmaceutical composition. The present therefore also relates to a pharmaceutical composition that comprises an inducer, vehicle, or monocyte or macrophage for restoring ICER, GPCR65 and/or GPCR132 expression levels or function. The pharmaceutical composition may be formulated with any known pharmaceutically acceptable carrier or diluent as well as any other known adjuvants and excipients in accordance with conventional techniques. The pharmaceutically acceptable carriers, diluents, adjuvants and excipients should be suitable for the chosen inductor of the present invention and the chosen mode of administration. A pharmaceutical composition of the present invention may also include diluents, fillers, salts, buffers, detergents (e. g., a nonionic detergent), stabilizers (e. g., sugars or protein-free amino acids), preservatives, tissue fixatives, solubilizers, and/or other materials suitable for inclusion in a pharmaceutical composition.

The pharmaceutical compositions of the present invention can be formulated by methods known to those skilled in the art. For example, such pharmaceutical compositions can be used parenterally, as injections which are sterile solutions or suspensions including the compositions along with water or another pharmaceutically acceptable liquid. For example, such compositions may be formulated as unit doses that meet the requirements for the preparation of pharmaceuticals by appropriately combining the compositions with pharmaceutically acceptable carriers, diluents, adjuvants or excipients, specifically with sterile water, physiological saline, a vegetable oil, emulsifier, suspension, surfactant, stabilizer, flavoring agent, excipient, vehicle, preservative, binder or such. In such preparations, the amount of active ingredient is adjusted such that an appropriate dose that falls within a pre-determined range can be obtained.

As will be apparent, the present invention is also applicable to methods for the treatment or prevention HF, and associated or resulting diseases such as ischemic heart failure IHF, myocardial infarction MI, HFrEF, or HFpEF using a herein described inductor of ICER, GPCR65 or GPCR132.

Lipid nanoparticles (LNPs) for non-viral delivery of ICER, GPCR65 or GPCR132 mRNA are preferred components of a pharmaceutical composition. In a preferred embodiment, the pharmaceutical composition comprises at least one LNP. In a preferred embodiment, the at least one LNP further comprises: i) at least one structural lipid; ii) at least one phospholipid; and iii) at least one PEGylated lipid.

In a further aspect, the at least one structural lipid is selected from cholesterol, fecosterol, fucosterol, beta sitosterol, sitosterol, ergosterol, campesterol, stigmasterol, brassicasterol, tomatidine, cholic acid, sitostanol, litocholic acid, tomatine, ursolic acid, alpha-tocopherol, Vitamin D3, Vitamin D2, Calcipotriol, botulin, lupeol, oleanolic acid, beta-sitosterol-acetate and any combinations thereof.

In some embodiments, the at least one phospholipid can be any one of 1,2-distearoyl-sn-glycero-3-phosphocholine (DSPC), 1,2-dioleoyl-sn- glycero-3-phosphoethanolamine (DOPE), 1,2-dilinoleoyl-sn-glycero-3- phosphocholine (DLPC), 1,2-dimyristoyl-sn-glycero-phosphocholine (DMPC), 1.2- dioleoyl-sn-glycero-3-phosphocholine (DOPC), 1,2-dipalmitoyl-sn-glycero-3-phosphocholine (DPPC), 1,2-diundecanoyl-sn-glycero-phosphocholine (DUPC), 1- palmitoyl-2-oleoyl-sn-glycero-3-phosphocho line (POPC), 1,2-di-O-octadecenyl-sn- glycero-3-phosphocholine (18:0 Diether PC), 1-oleoyl-2-cholesterylhemisuc cinoyl- sn-glycero-3-phosphocholine (OChemsPC), 1-hexadecyl-sn-glycero-3- phosphocholine (C16 Lyso PC), 1,2-dilinolenoyl-sn-glycero-3-phosphocholine, 1,2- diarachidonoyl-sn-glycero-3-phosphocholine, 1,2-didocosahexaenoyl-sn-glycero-3- phosphocholine, 1,2-diphytanoylsn-glycero-3-phosphoethanolamine (ME 16.0 PE), 1,2-distearoyl-sn-glycero-3-phosphoethanolamine, 1,2-dilinoleoyl-sn-glycero-3- phosphoethanolamine, 1,2-dilinolenoyl-sn-glycero-3-phosphoethanolamine, 1,2- diarachidonoyl-sn-glycero-3-phosphoethanolamine, 1,2-didocosahexaenoyl-sn- glycero-3-phosphoethanolamine, 1,2-dioleoyl-sn-glycero-3-phospho-rac-(1-glycerol) sodium salt (DOPG), sodium (S)-2-ammonio-3-((((R)-2-(oleoyloxy)-3-(stearoyloxy)propoxy)oxidophosphoryl)oxy)propanoate (L-α-phosphatidylserine; Brain PS), dimyristoyl phosphatidylcholine (DMPC), dimyristoyl phosphoethanolamine (DMPE), dimyristoylphosphatidylglycerol (DMPG), dioleoyl- phosphatidylethanolamine4-(N-maleimidomethyl)-cyclohexane-1-carboxylate (DOPE-mal), dioleoylphosphatidylglycerol (DOPG), 1,2-dioleoyl-sn-glycero-3- (phospho-L-serine) (DOPS), acell-fusogenicphospholipid (DPhPE), dipalmitoylphosphatidylethanolamine (DPPE), 1,2-Dielaidoyl-sn-phosphatidylethanolamine (DEPE), dipalmitoylphosphatidylglycerol (DPPG), dipalmitoylphosphatidylserine (DPPS), distearoylphosphatidylcholine (DSPC), distearoyl-phosphatidyl-ethanolamine (DSPE), distearoyl phosphoethanolamineimidazole (DSPEI), 1,2-diundecanoyl-sn-glycero- phosphocholine (DUPC), egg phosphatidylcholine (EPC), 1,2-dioleoyl-sn-glycero-3- phosphate (18:1 PA; DOPA), ammonium bis((S)-2-hydroxy-3-(oleoyloxy)propyl) phosphate (18:1 DMP; LBPA), 1,2-dioleoyl-sn-glycero-3-phospho-(1'-myo-inositol) (DOPI; 18:1 PI), 1,2-distearoyl-sn-glycero-3-phospho-L-serine (18:0 PS), 1,2-dilinoleoyl-sn-glycero-3-phospho-L-serine (18:2 PS), 1-palmitoyl-2-oleoyl-sn- glycero-3-phospho-L-serine (16:0-18:1 PS; POPS), 1-stearoyl-2-oleoyl-sn-glycero-3- phospho-L-serine (18:0-18:1 PS), 1-stearoyl-2-linoleoyl-sn-glycero-3-phospho-L- serine (18:0-18:2 PS), 1-oleoyl-2-hydroxy-sn-glycero-3-phospho-L-serine (18:1 Lyso PS), 1-stearoyl-2-hydroxy-sn-glycero-3-phospho-L-serine (18:0 Lyso PS), and sphingomyelin.

In a further aspect, the at least one PEGylated lipid is selected from (R)-2,3-bis(octadecyloxy)propyl-1- (methoxypoly(ethyleneglycol)2000)propylcarbamate, PEG-S-DSG, PEG-S-DMG, PEG-PE, PEG-PAA, PEG-OH DSPE C18, PEG-DSPE, PEG-DSG, PEG-DPG, PEG- DOMG, PEG-DMPE Na, PEG-DMPE, PEG-DMG2000, PEG-DMG C14, PEG-DMG 2000, PEG-DMG, PEG-DMA, PEG-Ceramide C16, PEG-C-DOMG, PEG-c-DMOG, PEG-c-DMA, PEG-cDMA, PEGA, PEG750-C-DMA, PEG400, PEG2k-DMG, PEG2k-C11, PEG2000-PE, PEG2000P, PEG2000-DSPE, PEG2000-DOMG, PEG2000-DMG, PEG2000-C-DMA, PEG2000, PEG200, PEG(2k)-DMG, PEG DSPE C18, PEG DMPE C14, PEG DLPE C12, PEG Click DMG C14, PEG Click C12, PEG Click C10, N(Carbonyl-methoxypolyethylenglycol-2000)-1,2-distearoyl-sn- glycero3-phosphoethanolamine, Myrj52, mPEG-PLA, MPEG-DSPE, mPEG3000-DMPE, MPEG-2000-DSPE, MPEG2000-DSPE, mPEG2000-DPPE, mPEG2000- DMPE, mPEG2000-DMG, mDPPE-PEG2000, l,2-distearoyl-sn-glycero-3- phosphoethanolamine-PEG2000, HPEG-2K-LIPD, Folate PEG-DSPE, DSPE- PEGMA 500, DSPE-PEGMA, DSPE-PEG6000, DSPE-PEG5000, DSPE-PEG2K- NAG, DSPE-PEG2k, DSPE-PEG2000maleimide, DSPE-PEG2000, DSPE-PEG, DSG-PEGMA, DSG-PEG5000, DPPE-PEG-2K, DPPE-PEG, DPPE-mPEG2000, DPPE-mPEG, DPG-PEGMA, DOPE-PEG2000, DMPE-PEGMA, DMPE-PEG2000, DMPE-Peg, DMPE-mPEG2000, DMG-PEGMA, DMG-PEG2000, DMG-PEG, distearoyl-glycerol-polyethyleneglycol, CI8PEG750, CI8PEG5000, CI8PEG3000, CI8PEG2000, CI6PEG2000, CI4PEG2000, C18-PEG5000, C18PEG, C16PEG, C16 mPEG (polyethylene glycol) 2000 Ceramide, C14-PEG-DSPE200, C14-PEG2000, C14PEG2000, C14-PEG 2000, C14-PEG, C14PEG, 14:0-PEG2KPE, 1,2-distearoyl- sn-glycero-3-phosphoethanolamine-PEG2000, (R)-2,3-bis(octadecyloxy)propyl-1-(ethoxypoly(ethyleneglycol)2000)propylcarbamate, (PEG)-C-DOMG, PEG-C- DMA, and DSPE-PEG-X. 82.

In a preferred embodiment, the LNP further comprises at least one additional lipid component selected from 1,2-di-O-octadecenyl- sn-glycero-3-phosphocholine (18:0 Diether PC), 1,2-dilinolenoyl-sn-glycero-3- phosphocholine (18:3 PC), Acylcarnosine (AC), 1-hexadecyl-sn-glycero-3- phosphocholine (C16 Lyso PC), N-oleoyl-sphingomyelin (SPM) (C18:I), N- lignoceryl SPM (C24:0), N-nervonoylshphingomyelin (C24:1), Cardiolipin (CL), I,2- bis(tricosa-10,12-diynoyl)-sn-glycero-3-phosphocholine (DC8-9PC), dicetyl phosphate (DCP), dihexadecyl phosphate (DCP1), 1,2-Dipalmitoylglycerol-3- hemisuccinate (DGSucc), short-chain bis-n-heptadecanoyl phosphatidylcholine (DHPC), dihexadecoyl-phosphoethanolamine (DHPE), 1,2-dilinoleoyl-sn-glycero-3- phosphocholine (DLPC), I,2-dilauroyl-sn-glycero-3-PE (DLPE), dimyristoyl glycerol hemisuccinate (DMGS), dimyristoyl phosphatidylcholine (DMPC), dimyristoyl phosphoethanolamine (DMPE), dimyristoylphosphatidylglycerol (DMPG), dioleyloxybenzylalcohol (DOBA), 1,2-dioleoylglyceryl-3-hemisuccinate (DOGHEMS), N-[2-(2-{2-[2-(2,3-Bis-octadec-9-enyloxy-propoxy)-ethoxy]-ethoxy}- ethoxy)-ethyl]-3-(3,4, 5-1 rihydroxy-6-hydroxymethyl-1etrahydro-pyran-2-ylsulfanyl)- propionamide (DOGP4αMan), dioleoylphosphatidylcholine (DOPC), dioleoylphosphatidylethanolamine (DOPE), dioleoylphosphatidylethanolamine4-(N- maleimidomethyl)-cyclohexane-1-carboxylate (DOPE-mal), dioleoylphosphatidylglycerol (DOPG), 1,2-dioleoyl-sn-glycero-3-(phospho-L-serine) (DOPS), acell-fusogenicphospholipid (DPhPE), dipalmitoylphosphatidylethanolamine (DPPE), dipalmitoylphosphatidylglycerol (DPPG), dipalmitoylphosphatidylserine (DPPS), distearoylphosphatidylcholine (DSPC), distearoyl-phosphatidyl- ethanolamine (DSPE), distearoyl phosphoethanolamineimidazole (DSPEI), 1,2- diundecanoyl-sn-glycero-phosphocholine (DUPC), egg phosphatidylcholine (EPC), histaminedistearoylglycerol (HDSG), 1,2-Dipalmitoylglycerol-hemisuccinate-Nα- Histidinyl-Hemisuccinate (HistSuccDG), N-(5'-hydroxy-3'-oxypentyl)-10-12- pentacosadiynamide (h-Pegi-PCDA), 2-[I-hexyloxyethyl]-2-devinylpyropheophorbide-a (HPPH), hydrogenatedsoybeanphosphatidylcholine (HSPC), 1,2-Dipalmitoylglycerol-O-α-histidinyl-Nα-hemisuccinate (IsohistsuccDG), mannosialized dipalmitoylphosphatidylethanolamine (ManDOG), I,2-Dioleoyl-sn- Glycero-3-Phosphoethanolamine-N-[4-(p-maleimidomethyl)cyclohexane- carboxamide] (MCC-PE), 1,2-diphytanoyl-sn-glycero-3-phosphoethanolamine (ME 16:0 PE), 1-myristoyl-2-hydroxy-sn-glycero-phosphocholine (MHPC), a thiol- reactive maleimide headgroup lipid e.g.1,2-dioleoyl-sn-glycero-3- phosphoethanolamine-N-[4-(p-maleimidophenyl)but-yramid (MPB-PE), Nervonic Acid (NA), sodium cholate (NaChol), I,2-dioleoyl-sn-glycero-3- [phosphoethanolamine-N-dodecanoyl (NC12-DOPE), 1-oleoyl-2-cholesteryl hemisuccinoyl-sn-glycero-3-phosphocholine (OChemsPC), phosphatidylethanolamine lipid (PE), PE lipid conjugated with polyethylene glycol(PEG) (e.g., polyethylene glycol-distearoylphosphatidylethanolamine lipid (PEG-PE)), phosphatidylglycerol (PG), partially hydrogenated soy phosphatidylchloline (PHSPC), phosphatidylinositol lipid (PI), phosphotidylinositol-4-phosphate (PIP), palmitoyloleoylphosphatidylcholine (POPC), phosphatidylethanolamine (POPE), palmitoyloleyolphosphatidylglycerol (POPG), phosphatidylserine (PS), lissamine rhodamineB-phosphatidylethanolamine lipid (Rh-PE), purifiedsoy- derivedmixtureofphospholipids (SIOO), phosphatidylcholine (SM), 18-1-trans-PE, 1- stearoyl-2-oleoyl-phosphatidyethanolamine (SOPE), soybean phosphatidylcholine (SPC), sphingomyelins (SPM), alpha, alpha-trehalose-6,6'-dibehenate (TDB), I,2- dielaidoyl-sn-glycero-3-phophoethanolamine (transDOPE), ((23S,5R)-3- (bis(hexadecyloxy)methoxy)-5-(5-methyl-2,4-dioxo-3,4-dihydropyrimidin-1(2H)-yl)tetrahydrofuran-2-yl)methylmethylphosphate, 1,2-diarachidonoyl-sn-glycero-3-phosphocholine, 1,2-diarachidonoyl-sn-glycero-3-phosphoethanolamine, 1,2-didocosahexaenoyl-sn-glycero-3-phosphocholine, 1,2-didocosahexaenoyl-sn-glycero- 3-phosphoethanolamine, 1,2-dilinolenoyl-sn-glycero-3-phosphocholine, 1,2- dilinolenoyl-sn-glycero-3-phosphoethanolamine, 1,2-dilinoleoyl-sn-glycero-3- phosphoethanolamine, 1,2-dioleyl-sn-glycero-3-phosphoethanolamine, 1,2-distearoyl- sn-glycero-3-phosphoethanolamine, 16-O-monomethyl PE, 16-O-dimethyl PE, and dioleylphosphatidylethanolamine.

In a preferred embodiment, the LNP comprises: (a) a PEG-lipid (b) a structural lipid; and (c) a non-ionizable lipid and/or a zwitterionic lipid.

In a further aspect, the lipid nanoparticle further comprises an additional ionizable lipid, preferably a PEG-lipid such as PEG-c-DOMG, PEG-DMG, PEG-DLPE, PEG-DMPE, PEG-DPPC, and PEG-DSPE.

The packaging of ICER, GPCR65 and/or GPCR132 nucleic acid within said LNPs and delivery to the target monocyte or macrophage cells can be achieved by known methods in the art.

The present invention is further illustrated in the following examples. By no means shall the present invention be limited to those specific examples. The invention also comprises the combination of embodiments or features as disclosed herein or as apparent to a person skilled in the art.

### Description of the Figures:

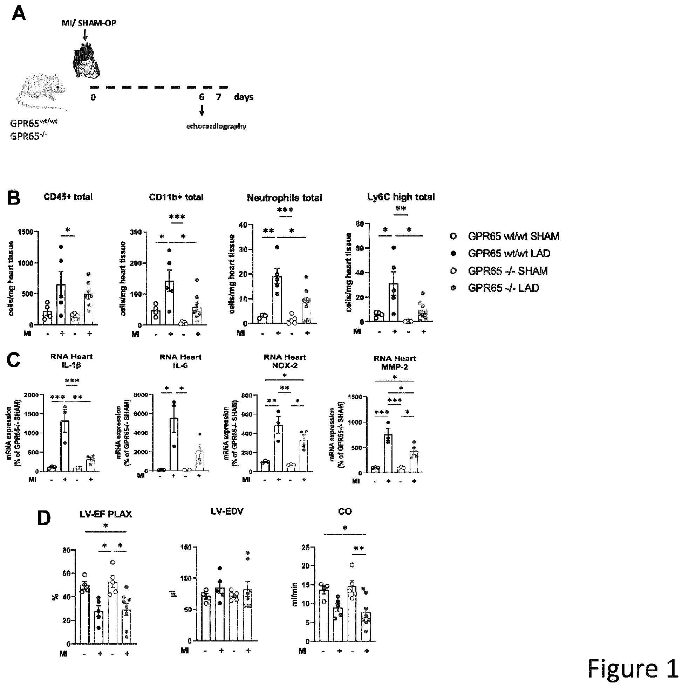

**Figure 1****:** (A) Study Scheme (B) Flow cytometry with quantification of CD45⁺ leukocytes, CD11b⁺ myeloid cells, Ly6G⁺ neutrophils and Ly6C^{high} proinflammatory monocytes in GPR65^{-/-} mice and WT littermates 7d after MI (C) Quantification of Real-Time PCR expression of interleukin 1beta (IL1ß), interleukin 6 (IL6), NADPH-Oxidase 2 (Nox2) and Matrix-Metalloprotease 2 (MMP-2) in GPR65^{-/-} mice and WT littermates 7d after MI. (D) Small-animal high frequency echocardiography of GPR65^{-/-}mice and WT littermates 6d after MI with quantification of LV-Ejection fraction, -enddiastolic volume and Cardiac Output.

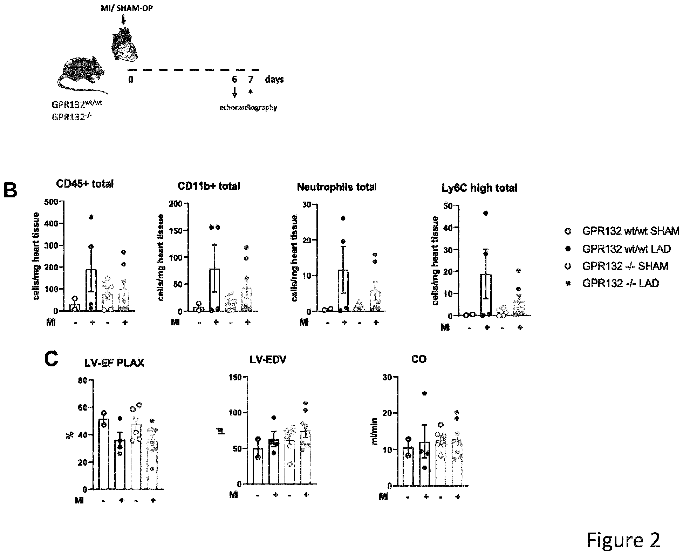

**Figure 2****:** (A) Study Scheme (B) Flow cytometry with quantification of CD45⁺ leukocytes, CD11b⁺ myeloid cells, Ly6G⁺ neutrophils and Ly6C^{high} proinflammatory monocytes in GPR132^{-/-} mice and WT littermates 7d after MI (C) Small-animal high frequency echocardiography of GPR132^{-/-} mice and WT littermates 6d after MI with quantification of LV-Ejection fraction, - enddiastolic volume and Cardiac Output

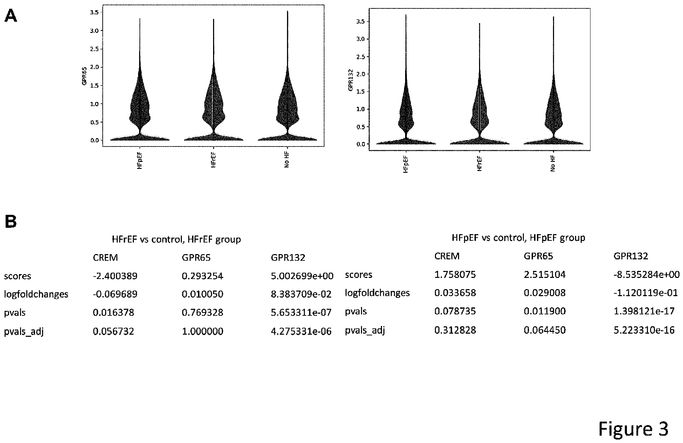

**Figure 3****:** (A) + (B) Single cell sequencing of circulating classical monocytes in heart failure patients with reduced ejection fraction (HFrEF) and preserved ejection fraction (HFpEF) and quantification of CREM, GPCR65 and GPCR132.

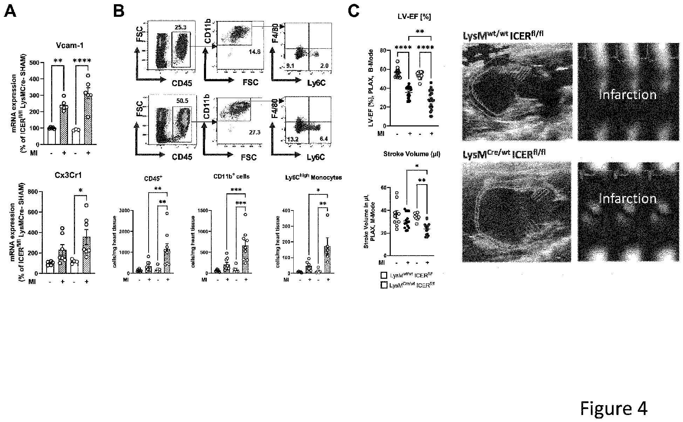

**Figure 4****:** (A) Cardiac expression of adhesion molecules with vascular adhesion molecule 1 (Vcam-1) and CX3C chemokine receptor 1 (Cx3Crl) as well as cardiac infiltration of CD45⁺ leukocytes, CD11b⁺ myeloid cells and Ly6C^{high} inflammatory monocytes 7 days after myocardial infarction (B) n=3-9 animals per group. (C) Small animal echocardiography on day 6 after MI with quantification of left ventricular ejection fraction (LV-EF) and left ventricular stroke volume quantification of LysM^{wt/wt} ICER^{flox/flox} and LysM^{Cre/wt} ICER^{flox/flox} mice 6 days after LAD ligation or sham surgery, n=12-23 mice per group.and representative images of MI animals (right). One-way ANOVA, *=p<0.05, **=p<0.01;***=p>0.001,****=p<0.0001.

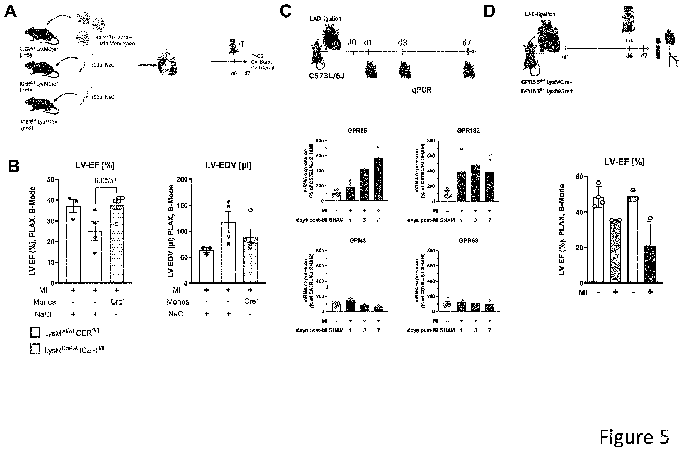

**Figure 5****:** Therapeutic potential of the GPR65-ICER signaling pathway in cardiovascular diseases. A) Overview of the study design of an adaptive monocyte transfer or NaCl (150µl) of ICER-competent WT monocytes in LysM^{Cre/wt} ICER^{flox/flox} experimental animals immediately before permanent LAD ligation. B) Small animal echocardiography on day 6 after MI with quantification of left ventricular ejection fraction (LV-EF) and left ventricular end-diastolic volume (LV-EDV), n=3-5 C) Study design of C57BL6J mice in the LAD ligation model and real-time PCR analyses of infarcted heart tissue 1, 3, 7 days after MI or SHAM with quantification of GPR65, GPR132, GPR4 and GPR68 n=1-9 mice per group. D) Study design of LysM^{wt/wt} GPR65^{flox/flox} and LysM^{Cre/wt} GPR65^{flox/flox} LAD ligation or sham surgery with small animal echocardiography and quantification of LV EF on day 6, n=2-4. One-way ANOVA, *=p<0.05, **=p<0.01,***=p>0.001,****=p<0.0001.

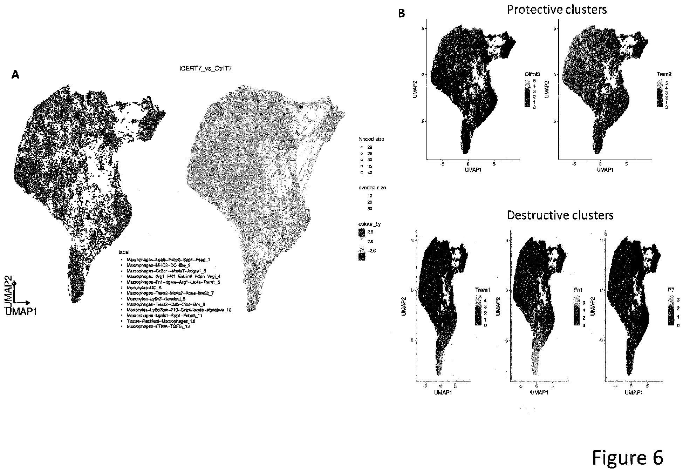

**Figure 6****:** Single-cell sequencing of immune cells in the infarcted myocardium. A) CD45 (cluster of differentiation 45)+ single-cell data sets published by Haghverdi et al (steady state before myocardial infarction [MI], days 1, 3, 5, and 7 post-MI) was integrated with our data set from day 7 post-MI obtained from LysM^{wt/wt} ICER^{flox/flox} and LysM^{Cre/wt} ICER^{flox/flox} mice using the Fastmnn from the Batchelor package in R. Left: Uniform Manifold Approximation and Projection (UMAP) of the different monocytes and macrophages in the infarcted myocardium, right: Visualization of differential representation of the different macrophage subpopulations in LysM^{Cre/wt} ICER^{flox/flox} mice in comparison to WT controls 7 days post MI. B) Visualization of protective and destructive macrophage clusters in the ischemic myocardium using the surface markers Trem2, Ofml3 (protective) and Trem 1, Fn1 and F7 (destructive).

### Examples:

The inventors discovered that mice with a global knockout of GPCR65 (GPR65^{-/-}) and GPCR132 (GPR132^{-/-}) exhibit reduced cardiac infiltration of pro-inflammatory immune cells like CD11b+ myeloid cells, Ly6G+ neutrophils or Ly6C^{high} proinflammatory monocytes in a model of myocardial infarction due to ligation of the left anterior descending artery (LAD). This was combined with reduced expression of pro-inflammatory cytokines like interferon 1 beta (IL1b) or TNF alpha as well as less expression of the ROS-producing enzyme NADPH-oxidase 2 (Nox2) or the profibrotic enzyme MMP2 quantified by real-time PCR analysis. The phenotype was more pronounced in the GPR132^{-/-} compared to GPR65^{-/-} mice **(****Figure 1A-C** **and** **2 A-B).** Cardiac function was only slightly altered at d7 post MI **(****Figure 1D** **and** **2C).**

Next, monocytes were analyzed from patients with heart failure from the MyoVasc cohort²⁸. Here it could be shown that GPCR65 and GRCP132 are differentially expressed in classical monocytes in heart failure patients with reduced ejection fraction (HFrEF) and preserved ejection fraction (HFpEF) **(****Figure 3A-B**

The data suggest that modulation of the GPCR-coupled acidic stress response involving the induction of ICER has the potential to attenuate post-MI cardiac remodeling as well as vascular protection, prevent development of ischemic heart failure and vascular dysfunction and maintain tissue homeostasis in case of nutrient/oxygen deprivation and acidification in cardiovascular disease.

To address the importance of metabolic signaling pathways and the modulatory role of ICER on these immune cells after acute myocardial infarction and in arterial hypertension, mice with a conditional knockout of ICER on myeloid cells (LysM^{Cre/wt} ICER^{fl/fl}), as well as with a conditional knockout of the superordinate G-protein coupled receptor GPCR65 (LysM^{Cre/wt} GPR65^{fl/fl}) lacking the pH sensing mechanisms upstream of ICER, were used **(****Figure 4****+5).** As suggested by the data, tissue acidification leads to an activation of ICER in macrophages and macrophages from ICER- deficient mice are pro-inflammatory. Accordingly, elevated cAMP levels attenuate the inflammatory function of monocytes and macrophages. Further experimental studies have shown that they promote the development of malignant cardiac arrhythmias and heart failure.

Interestingly, in LysM^{Cre/wt} ICER^{fl/fl} mice with chronic myocardial ischemia, increased cardiac expression of myeloid adhesion molecules such as vascular adhesion molecule 1 (Vcam-1) and CX3C chemokine receptor 1 (Cx3Crl) as well as increased infiltration of CD11b+ myeloid cells, especially pro-inflammatory Ly6C^{high} monocytes, into the ischemic myocardium could be observed when ICER cannot be expressed in myeloid cells. As a result, cardiac function was impaired with reduced left ventricular ejection fraction (LV-EF). This clearly demonstrates that blocking the intrinsic metabolic acid stress response system enhances pro-inflammatory myeloid cell immunity, which is detrimental to cardiovascular function and survival. Single-cell RNA analyses of cardiac CD45⁺ cells revealed differential macrophage polarization due to a pro-inflammatory phenotype and a differential expression of acidosis response genes and G protein-coupled receptors such as GPR65 in these macrophage populations. Thus, these data strongly suggest that circulating and infiltrating immune cells connect the functional systems and that targeted modulation of this stress response system, for example by temporally and locally restricted induction of ICER expression, represents a novel, effective therapeutic option.

The therapeutic potential has furthermore been demonstrated by in vivo experiments that involved the isolation of bone marrow-derived monocytes from donor animals in which the myeloid cells are ICER-competent (LysM^{wt/wt} ICER^{flox/flox}, referred to as wildtype controls) and transfer into recipient animals specifically lacking ICER in lysozyme M-positive myeloid cells (LysM^{Cre/wt} ICER^{flox/flox}). Cell transfer was performed from 1 million monocytes isolated from the bone marrow immediately prior to surgical myocardial infarction induction and dissolved in 150µl NaCl injected intravenously into the tail vein. The study design consisted of a total of three groups, all of which underwent experimental, non-reperfused myocardial infarction by permanent ligation of the LAD:
Group 1: LysM^{Cre/wt} ICER^{flox/flox} transfer of 1 Mio monocytes from LysM^{wt/wt} ICER^{flox/flox} animals into 150µl NaCl.
Group 2: LysM^{Cre/wt} ICER^{flox/flox} injection with 150µl NaCl and
Group 3: LysM^{wt/wt} ICER^{flox/flox} injection with 150µl NaCl **(****Figure 5A****).**

The invention demonstrates that the transfer of ICER-competent monocytes could re-instate the cardiac phenotype of the wildtype with an improvement of the left ventricular ejection fraction **(****Figure 5B****).** In contrast, ICER deficiency in the previous studies led to a significant deterioration of cardiac function associated with an increased immigration of myeloid immune cells **(****Figure 4B and** C). To better understand the signaling pathway of metabolic acid stress on immune cells, RT-PCR analyses from infarcted heart tissue were performed at defined time points after a myocardial infarction (after 1, 3 or 7 days). In the mouse model of non-reperfused myocardial infarction, an upregulation of GPR65 and GPR132 could be detected **(****Figure 5C****).**

Complementary experiments were performed in mice with a deficiency of GPR65 on myeloid immune cells (LysM^{Cre/wt} GPR65^{flox/flox_}) and LysM^{wt/wt} GPR65^{flox/fox}mice as wildtype controls. These were also in the non-reperfused MI model by LAD ligation (as well as SHAM controls) over a follow-up period of 7 days and showed a phenotype similar to myeloid cell-ICER knockout with worsened LV function **(****Figure 5D****).**

In addition, a further analysis of the single-cell RNAseq data of cardiac leukocytes from ICER knockout or wildtype mice was carried out in order to be able to make statements about differentially regulated genes in the individual myeloid cell clusters in the heart tissue after non-reperfused MI. Therefor single-cell data sets published by Haghverdi et al (steady state before MI and days 1, 3, 5, and 7 post-MI) were integrated with the data set from day 7 post-MI obtained from LysM^{wt/wt} ICER^{flox/flox} and LysM^{Cre/wt} ICER^{flox/flox} mice using the Fastmnn from the Batchelor package in R. Here it could be clearly shown that ICER-deficient mice lack especially protective and reparative macrophage cluster 7 days post MI (cluster 7 and 9) with a prolonged pro-inflammatory phenotype **(****Figure 6****).**

The present invention provides clear evidence that the cellular mechanisms for sensing metabolic acid stress by GPCR65, GPCR132 or ICER, are crucial for initiating reparative processes after a non-reperfused MI. If these mechanisms are missing, this leads to a prolonged, excessive immune response with maladaptive cardiac remodeling due to a lack of reparative mechanisms. This is a novel and promising therapeutic approach that has not been investigated or described in the context of cardiovascular diseases and heart failure development, prevention and treatment, especially after myocardial infarction.

### Material & Methods:

### 1. Animals/ mouse strains

All experiments were carried out on male and female mice aged 9 to 14 weeks. The experimental animals were housed and bred by the research group of Prof. Dr. Tobias Bopp (Research Center for Immunotherapy (FZI), University Medical Center Mainz). The genetic background is C57BL/6. Experimental animals of the GPR65 and GPR132 experiments were originally obtained from The Jackson Laboratory (ME, USA). The experimental animals of the ICER experiments were originally a gift from Shogo Endo (Aging Neuroscience Research Team, Tokyo Metropolitan Institute of Gerontology) and Nobuhiko Kojima (Department of Life Sciences, Toyo University) to the research group of Prof. Dr. Tobias Bopp. All animal experiments were conducted in compliance with the German Animal Welfare Act (TSchG) and the Animal Welfare Experimental Animal Ordinance (TierSchVersV) and approved by the Rhineland-Palatinate State Investigation Office in animal experiment application numbers G 20-1-051 and G24-1-008.

### Mouse lines:

C57BI/6J: Wildtype mice
B6.129X1-Gpr65tm1Witt/J: The mice have a homozygous mutation in the Gpr65 gene. The expression of the gene product is prevented by in vitro substitution of a DNA sequence of exon 2 (targeted mutation; knockout). Abbreviation: GPR65-/- (control: GPR65wt/wt)
B6.129X1(C)-Gpr132tm1Witt/J: The mice have a homozygous mutation in the Gpr132 gene. Replacement of 98 % of exon 2 with a specific vector prevents expression of the gene product (GPR132, G2A). Abbreviation: GPR132 -/- (control: GPRR132wt/wt)
57BL/6J Gpr65em1Bopp, Lyz2tm1(cre)Ifo: The mouse strain has a conditional knockout of GPCR 65 on all myeloid cells. GPR65 can perceive the extracellular pH value and thereby activate intracellular processes, especially via cAMP changes. GPR65 reduces immune-mediated inflammation by regulating cytokine production by T cells and macrophages. The mouse line was generated by Prof. Bopp's research group and has not yet been published. Abbreviation: LysMCre/wt GPR65fl/fl (control: LysMwt/wt GPR65fl/fl)
Cremfl/fl Lyz2-Cre: The mouse strain exhibits a selective knockout of the ICER gene in myeloid cells and is created by crossing Lyz2-Cre mice with Cremfl/fl mice. The Cre-loxP system is used for this. In Lyz2-Cre test animals, the expression of Cre recombinase is subject to the transcriptional control of the lysozyme 2 gene (Lyz2), which is predominantly expressed by macrophages and partly also by neutrophil granulocytes. In Cremfl/fl mice, the intrinsic P2 promoter of the Crem gene locus, which is essential for ICER expression, is flanked by loxP sites. Expression of the Cre recombinase in the crossed mouse strain (Cremfl/flLyz2-Cre) results in excision of the ICER promoter. Thus, ICER expression is selectively prevented in myeloid cells. The gene expression of other CREM isoforms remains unaffected. Abbreviation: LysMCre/wt ICERfl/fl (control: LysMwt/wt ICERfl/fl)

In order to analyse the role of pH-dependent signaling pathways after acute myocardial infarction, the above-mentioned mice and corresponding control animals were examined according to a standardized study scheme. At the beginning of the studies, the experimental animals underwent surgery. The animals in the experimental group underwent surgical induction of myocardial infarction by ligation of the anterior interventricular ramus (RIVA, LAD: left anterior descending artery). The animals in the control group underwent a SHAM surgery.

Postoperatively, the effects of the surgery on cardiovascular function were examined in more detail at defined time points. This included quantification of left ventricular function by echocardiography and immune cell infiltration of various organs by flow cytometry. To analyze the processes at the cellular level, the expression of various target genes was examined using polymerase chain reaction (RT-PCR). In addition, single cell analysis of cardiac leukocytes was performed by RNA sequencing.

In study 4, monocytes from animals of the ICER-wt group (LysMwt/wt ICERfl/fl) were transferred to the LysMCre/wt ICERfl/fl animals via tail vein injection immediately before the induction of AMI. In the control group, an NaCl solution was injected.

Animals in an experimental group were kept in common cages where possible.

| | |
|---|---|
| Study 1 | GPR65 wt/wt and -/- (SHAM vs. MI, 7d) |
| Study 2 | GPR132 wt/wt and -/- (SHAM vs. MI, 7d) |
| Study 3 | LysMCre/wt ICERfl/fl (control: LysMwt/wt ICERfl/fl) (SHAM vs. MI, 7d) |
| Study 4 | LysMCre/wt ICERfl/fl (control: LysMwt/wt ICERfl/fl) (MI, 7d): Adaptive Monocyte Transfer (d0) |
| Study 5 | C57BI/6J (SHAM vs. MI d1,3,7) |
| Study 6 | LysMCre/wt GPR65fl/fl (control: LysMwt/wt GPR65fl/fl) (SHAM vs. MI, 7d) |

### 2. Myocardial infarction induction by permanent ligation of the LAD

The dosage of anaesthetic drugs was weight-adapted with medetomidine (500 µg/kg body weight), fentanyl (50 µg/kg body weight) and midazolam (5 mg/kg body weight). To prevent eye damage due to dehydration, Bepanthen eye ointment was applied for the duration of the anaesthesia. Adequate depth of anaesthesia was ensured by assessing the interdigital reflex. Prior to surgery, the surgical field was cleared of hair using an electric razor and depilatory cream and then cleaned with skin disinfectant. The test animals were placed in the supine position on the warming plate and secured with a piece of fixation plaster on each of the four extremities and on the tail. To facilitate intubation, the incisors of the upper jaw were wrapped with dental floss and fixed to the plate with slight cranial traction. For the duration of intubation, the warming plate was placed in a 30° head-up position and a flexible cold light was directed onto the neck area of the mouse from the outside. Furthermore, the tongue was mobilized with atraumatic forceps and fixed laterally. To achieve an optimal view of the vocal cord level, the lower jaw was raised slightly with a laryngoscope. With good visibility, intubation was performed with a size 22 G intravenous catheter. The plastic attachment of the intravenous catheter was then cut off with scissors and the catheter was connected to the ventilator (MiniVent Ventilator, Hugo Sachs Elektronik). Ventilation was performed with an air mixture of room air and oxygen (70-80 ml/min) at a tidal volume of 200 µl and a ventilation rate of 150/min.

Surgical induction of myocardial infarction was performed according to a modified and standardized protocol by Tarnavski.1 Under microscopic view, an 8-10 mm long skin incision was first made at the level of the third to fourth intercostal space along the course of the ribs from the left anterior axillary line to parasternal. After blunt detachment of the skin from the subcutaneous fatty tissue, the muscle layers were gradually separated. The thorax was opened between the fourth and fifth ribs, sparing the surrounding vessels as well as the heart and lungs. The intercostal space was spread to a size of 6-8 mm by looping a Prolene suture of 6-0 thickness around the upper and lower ribs. The sutures were fixed to the table for this purpose. This was followed by blunt opening of the pericardium. With a view of the left auricle and left ventricle, the LAD was then located under microscopic magnification and wrapped with an 8-0 Prolene suture approximately 1.5 mm below the left auricle and mechanically ligated by subsequent knotting. Successful ligation of the LAD was indicated by immediate fading of the distal myocardium (especially the apex of the heart) as well as any wall motion and cardiac arrhythmias that occurred. After successful ligation of the LAD, the sutures were removed to spread the ribs and a multi-layer wound closure was performed using a 6-0 Prolene suture. For this, the thorax was first closed by suturing the intercostal muscles, followed by suturing the pectoral muscles and finally the skin suture with subsequent disinfection of the surgical field. The mice in the control group (SHAM-OP) were subjected to the same protocol with the exception of the LAD ligation; thus, after opening the pericardium, the wound was closed without further manipulation.

Immediately after skin closure, an intraperitoneal injection of an analgesic (buprenorphine 0.075 mg/kg body weight, s.c.) and an antidote mixture consisting of atipamezole (0.05 mg/kg body weight) and flumazenil (0.01 µg/kg body weight) was administered. With sufficient spontaneous respiration and recurring strong inter-toe reflex, the animals were extubated and placed in a fresh cage. On the first and second postoperative day, s.c. buprenorphine was repeated twice daily. The general condition of the operated mice was subsequently evaluated daily according to a standardized scoring scheme (stress score) and documented.

### 3. High-frequency small animal echocardiography

To evaluate cardiac function, transthoracic echocardiography was performed on the sixth postoperative day using the Visual-Sonics small animal ultrasound device (Vevo 3100 Ultrasound System) with a 20-46 MHz transducer (MX400, Fujifilm, Canada).2,3 The examination was performed under isoflurane inhalation anaesthesia. For induction of anaesthesia, the test animals were placed individually in a chamber that was gassed with a mixture of oxygen and 2.5 % isoflurane. Anesthesia was maintained by applying an isoflurane-oxygen mixture with 1.0 - 1.5 % isoflurane via a funnel covering the snout. Eye ointment was applied to protect the eyes opened under anesthesia. The mouse was placed supine on a warming plate (37 °C) with a piece of fixation plaster on each of the four extremities and on the tail. For temperature management, irradiation with a red-light lamp and continuous temperature measurement via a rectal probe were also performed. An electrocardiogram was recorded via electrodes integrated into the plate. Contact was established by means of electrode gel on the extremities. To optimize the examination conditions, the thorax was depilated with depilatory cream and ultrasound gel was then applied. The ultrasound images were then acquired while continuously monitoring the heart rate, respiratory rate and body temperature. Two different axes were examined in B-mode (brightness mode) and M-mode (motion mode). Direct and indirect measurements were then performed on the ultrasound images obtained and the left ventricular ejection fraction (LV-EF, in %), the left ventricular end-diastolic volume (LV-EDV) and the left ventricular cardiac output (CO) and stroke volume (SV) were calculated.

### 4. Flow cytometric examination of cardiac tissue

After euthanizing the animals, the heart was immediately removed and flushed with Krebs-Hepes buffer. The infarcted part of the heart was then cut out under the microscope, weighed and used immediately for flow cytometric analysis. The tissue was first minced manually using a razor blade. The minced tissue was taken up in 1 ml collagenase-DNase mix and incubated on a thermal mixer (30 min, 37 °C, 500 rpm). After 10 and 20 minutes, manual mixing was also carried out by swirling the reaction vessels. After 30 minutes, the suspension was pressed through a cell sieve (pore size 70 µm) into a 50 ml centrifuge tube. The digestion reaction was stopped by rinsing with 5 ml PBS/FCS. After centrifugation (6 min, 300 G, 4 °C) and subsequent removal of the supernatant, erythrocytes were lysed by incubation with 500 µl ACK lysis buffer (1 min, room temperature). This was stopped by adding 3 ml PBS/FCS. After renewed centrifugation and discarding the aqueous supernatant, the remaining cell pellet was dissolved in 200 µl PBS/FCS. After counting the contained cells using the Spark^{®} Multimode Microplate Reader from TECAN (Männedorf, CH), the entire suspension was stained with FACS antibodies. First, a 10-minute incubation in 50 µl Fc block (diluted 1:100, 10 min, 4 °C, dark) was performed. After a single wash with 100 µl PBS/FCS (centrifugation 6 min, 300 G, 4 °C), the samples were incubated with 50 µl of the antibody mixture for 30 min (4 °C, dark). After washing again, the pellets were resuspended in 200 µl PBS/FCS. This was followed by flow cytometric analysis of the fluorescently labeled cell suspensions. The following antibodies and surface antigens were used for staining:

| | |
|---|---|
| CD45 | (color: APC-eFluor 780; eBioscience, clone 30-F11) |
| B220 | (color: FITC; eBioscience, clone RA3-6B2) |
| CD90. 2 | (color: SuperBright 645; eBioscience, clone 53-2.1) |
| NK1.1 | (color: PE-Cy7; eBioscience, clone PK136) |
| CD11b | (color: FITC or PerCP-Cy5.5; eBioscience, clone M1/70) |
| Ly6G | (color: PE; BD Bioscience, clone 1A8) |
| Ly6C | (color: Pacific Blue; eBioscience, clone HK1.4) |
| F4/80 | (color: APC; eBioscience, clone BM8) |
| Viability Dye | (color: efluor 506; eBioscience) |

Cells were investigated using the AttuneTM NxT Flow Cytometer (Thermo Scientific, Germany) and subsequently analyzed using FlowJo software (FlowJo Version 10, BD, USA).4,5

### 5. Cardiac RNA analysis

RNA isolation was performed according to a modified protocol of acidic guanidinisothiocyanate-phenol extraction according to Chomczynski and Sacchi. First, the tissue was taken up together with a 5 mm steel ball in a 2 ml Eppendorf tube in 600 µl guanidine isothiocyanate buffer (Git buffer). This was followed by mechanical disruption using the Tissue Lyser II (Qiagen, Hilden, Germany) by crushing for 5 minutes at a frequency of 30 Hz, followed by lysis at -20 °C for at least one hour. The nucleic acid extraction was carried out in the next step by adding 600 µl of water-saturated phenol. As DNA and RNA can only be separated in an acidic environment, the sample was acidified by adding 60 µl sodium acetate (2M, pH 4.0). After thorough mixing using a vortexer, 300 µl of a chloroform-isoamyl alcohol mixture (24:1) was then added, resulting in the formation of a second phase. After incubation on ice for 15 minutes, the samples were centrifuged at 4 °C and 16,000 G for 20 minutes. The upper phase was transferred to a new 1.5 ml Eppendorf tube, then an equivalent volume of ice-cooled isopropanol was added. Precipitation was carried out at -20 °C for at least one hour. After renewed centrifugation (20 min, 16,200 G, 4 °C), the supernatant was removed and the resulting pellet was resuspended in 80 % ethanol. This was followed by a further centrifugation (10 min, 16,200 G, 4 °C). The supernatant was again removed and the remaining pellet was dried at room temperature for 5-10 min and then dissolved in RNase-free water. The dissolved RNA was stored at -80 °C until further use.

To determine the RNA concentration, the samples were first gently thawed (30 min at 4 °C, 5 min at room temperature, 4 min at 56 °C) and then stored on ice. The RNA concentration was measured by spectrophotometric determination of the optical density against a blank value (water). The Spark^{®} Multimode Microplate Reader (TECAN, Erlangen, Germany) was used for this purpose in the present study.

Real-time PCR (RT-PCR, also known as quantitative PCR [qPCR]) was used to quantify the RNA expression of various of the genes mentioned below in the heart. TaqMan probes based on the fluorescence resonance energy transfer method (FRET method) were used for this purpose. These are short oligonucleotides complementary to the target sequence, which are coupled at the 5' end with a reporter fluorophore and at the 3' end with a quencher.

For RT-PCR, the "QuantiTectTM Probe RT-PCR Kit" from Quiagen (Hilden, Germany) was used as a one-step RT-PCR. The primers and probes were purchased as "TaqMan^{®} Gene Expression Assays" from Applied Biosystems (Darmstadt, Germany). The preparation volume per well was 10 µl and was composed as follows:

### Reaction mixture:

| | |
|---|---|
| 2x QuantiTect Probe RT-PCR Master Mix | 5 µl |
| RNase free water | 1.9 µl |
| Primer 1 (TBP) | 0.5 µl |
| Primer 2 | 0.5 µl |
| QuantiTectProbeRT-Mix | 0.1 µl |

| | |
|---|---|
| Sample | 2 µl |

### RT-PCR program:

### Procedure Temperature Duration

1. reverse transcription 50°C 30 min
2. initial heat activation 95°C 15 min
3. primer hybridization 94°C 15 sec
4. elongation 60°C 1 min

A total of 50 PCR cycles were performed (steps 3. and 4.).

RT-PCR was performed using the CFX96 Real-Time PCR Detection System from BioRad (Munich, Germany). The ubiquitously expressed "TATA-box-binding protein" (TBP), which is not subject to gene regulation, served as the reference gene. Expression differences were calculated using the ΔΔCt method. In the evaluation, the RNA expression of the control group (wild-type experimental animals, SHAM-operated) was defined as 100 %. The RNA expression of the experimental groups was compared as a percentage.6,7

The following primers from the TaqMan^{®} Gene Expression Assay from Applied Biosystems (Thermo Fisher Scientific (Waltham, MA, USA)) were used for RT-PCR:

| **Primers** | | **Further descriptions** | **Gen** |
|---|---|---|---|
| Cx3cr1 | C-X3-C Motif Chemokine Receptor 1 | CCRL1, CMKBRL1, CMKDR1, GPR-13, GPRV28, V28 | CX3CR1 |
| IL-1ß | Interleukin 1 Beta | IL-1beta, IL-1b, RP23-384K11.2 | IL1B |
| IL-6 | Interleukin 6 | IL6, IFNB2 | IL6 |
| MMP-2 | Matrix Metalloproteinase 2 | MMP-II, Gelatinase A, CLG4A | MMP2 |
| NOX-2 | NADPH Oxidase 2 | Nox2, Cybb, GP91-PHOX | CYBB |
| VCAM-1 | Vascular Cell Adhesion Molecule 1 | CD106, Vcam1, Incam-100 | VCAM1 |
| GPR65 | G Protein-Coupled Receptor 65 | GPR65, TDAG8, HTDAG8 | GPR65 |
| GPR132 | G Protein-Coupled Receptor 132 | GPR132, G2A | GPR132 |
| GPR 4 | G Protein-Coupled Receptor 4 | GPR4, GPR6C.L | GPR4 |
| GPR 68 | G Protein-Coupled Receptor 68 | GPR69, ORG1 | GPR68 |
| TBP (FAM markiert) | TATA-Box Binding Protein | TBP1, GTF2D1, TFIID, Gtf2d, SCA17 | TBP |

### 6. Single-cell sequencing analysis (sqRNA) from cardiac tissue

CD45⁺ cells were isolated from infarcted myocardium by enzymatic digestion with the Multi Tissue Dissociation Kit 2 (#130-110-203, Miltenyi Biotec) using the gentle MACS dissociator (#130-093-235, Miltenyi Biotec). After homogenization, the suspensions were passed through a 70-µm cell strainer and resuspended in cell isolation buffer. Dead cells were removed using dead cell removal microspheres (No. 130-090-101, Miltenyi Biotec), followed by positive selection for anti-CD45 microspheres (No. 130-052-301, Miltenyi Biotec). The number of viable cells was measured using the automated cell counter of the BD Rhapsody platform. Samples that passed the viability assay were labelled to identify the biological replicates after pooling and sequencing. After washing the labelled cells, we repeated the cell counting and viability assay and loaded up to 30,000 cells onto a cartridge to achieve single cell isolation. The recovered RNA was first transcribed into complementary DNA (cDNA), followed by library preparation and quality control according to the Rhapsody pipeline. The finished single-cell libraries were sent for next-generation sequencing. Raw sequencing reads from the Illumina NovaSeq 6000 (Novogene, Cambridge, UK) were pre-processed according to the Illumina standard protocol. The sequence reads were trimmed to adapter sequences and further processed using Qiagen's CLC Genomics Workbench software (v21.0.5 with the CLC default settings for RNA-Seq analysis). The original single-cell matrices were analyzed using the established Pipeline of out working group.4

### 7. Adaptive cell transfer of monocytes

The "EasySep^{™} Mouse Monocyte Isolation Kit" (StemCell Technologies, Vancouver, Canada) was used to isolate monocytic cells from the bone marrow of the donor animals. First, the entire bone marrow was isolated from LysMCre/wt ICERfl/fl and LysMwt/wt ICERfl/fl mice from both upper and lower leg bones.8 The isolated bone marrow was dissolved in 1 ml EasySepTM buffer (StemCell Technologies, Vancouver, Canada) and then pressed through a cell sieve (pore size 70 µm) into a 50 ml centrifuge tube with a further 5 ml buffer and centrifuged (6 min, 300 G, 4 °C). After removing the supernatant, the cells were resuspended in 500 µl buffer and an Fc receptor block (EasySep^{™} Mouse FcR Blocker, StemCell Technologies, Vancouver, Canada; 100 µl per milliliter sample) was added. The samples were then transferred to a 5 ml round-bottom tube. In the next step, the EasySepTM antibody cocktail (StemCell Technologies, Vancouver, Canada) was prepared according to the manufacturer's instructions and added to the bone marrow suspension (100 µl per milliliter of sample). This contains antibodies against the cell surface markers of the non-monocytic cells to be removed. The antibodies were mixed with the cell suspension by careful pipetting. After incubation (5 min, 4 °C), EasySepTM Dextran RapidSpheresTM (StemCell Technologies, Vancouver, Canada) were added (75 µl per milliliter of sample) and mixed well with the sample suspension by careful swirling and pipetting. To enable stable binding of the magnetic particles to the antibody-labelled cells, a further incubation period followed (3 min, 4 °C). The cell suspension was then made up to 2.5 ml with buffer solution, carefully mixed and incubated in the EasySepTM Magnet for 3 minutes at room temperature. The labelled cells are attracted to the tube wall by the magnetic particles, while the unlabelled monocytes remain in the suspension. In the next step, the monocyte-rich suspension was transferred to a new 5 ml round-bottom tube in a liquid movement. To increase the purity of the isolated monocytes, a further separation step followed in the EasySepTM magnet (3 min, room temperature). The purified monocyte fraction was again transferred in a liquid movement into a 5 ml tube. 10 µl of the sample was then stained with 90 µl trypan blue in order to count the purified monocyte cells using the Spark^{®} Multimode Microplate Reader from TECAN (Männedorf, CH).

The test animals were injected once with 1 x 106 monocytic cells immediately before the surgical induction of myocardial infarction. For this purpose, an appropriate volume was removed from the monocyte suspension, which was then centrifuged (6 min, 300 G, 4 °C) and resuspended in 150 µl 0.9% NaCl solution. For tail vein injection, the animals were fixed in restrainers and the monocyte solution was transferred via a 1 ml syringe with a 26 G cannula attached. The two control groups (wild-type control: LysM^{wt/wt} ICERfl/fl negative control: LysMCre/wt ICERfl/fl) were injected with 150 µl isotonic saline solution instead of the monocyte suspension.

### Non-patent literature:

1. Hong KS, Saver JL. Quantifying the value of stroke disability outcomes: WHO global burden of disease project disability weights for each level of the modified Rankin Scale. Stroke. 2009;40:3828-3833.
2. Meier T, Grafe K, Senn F, Sur P, Stangl GI, Dawczynski C, Marz W, Kleber ME, Lorkowski S. Cardiovascular mortality attributable to dietary risk factors in 51 countries in the WHO European Region from 1990 to 2016: a systematic analysis of the Global Burden of Disease Study. EurJ Epidemiol. 2019;34:37-55.
3. Authors/Task Force M, Piepoli MF, Hoes AW, Agewall S, Albus C, Brotons C, Catapano AL, Cooney MT, Corra U, Cosyns B, Deaton C, Graham I, Hall MS, Hobbs FD, Lochen ML, Lollgen H, Marques-Vidal P, Perk J, Prescott E, Redon J, Richter DJ, Sattar N, Smulders Y, Tiberi M, van der Worp HB, van Dis I, Verschuren WM, Additional Contributor: Simone B, Document R, De Backer G, Roffi M, Aboyans V, Bachl N, Bueno H, Carerj S, Cho L, Cox J, De Sutter J, Egidi G, Fisher M, Fitzsimons D, Franco OH, Guenoun M, Jennings C, Jug B, Kirchhof P, Kotseva K, Lip GY, Mach F, Mancia G, Bermudo FM, Mezzani A, Niessner A, Ponikowski P, Rauch B, Ryden L, Stauder A, Ture G, Wiklund O, Windecker S, Zamorano JL. 2016 European Guidelines on cardiovascular disease prevention in clinical practice: The Sixth Joint Task Force of the European Society of Cardiology and Other Societies on Cardiovascular Disease Prevention in Clinical Practice (constituted by representatives of 10 societies and by invited experts): Developed with the special contribution of the European Association for Cardiovascular Prevention & Rehabilitation (EACPR). Eur J Prev Cardiol. 2016;23:NPI-NP96.
4. Alie N, Eldib M, Fayad ZA, Mani V. Inflammation, Atherosclerosis, and Coronary Artery Disease: PET/CT for the Evaluation of Atherosclerosis and Inflammation. Clin Med Insights Cardiol. 2014;8:13-21.
5. Blankstein R, Libby P, Bhatt DL. Arterial Inflammation: The Heat Before the Storm. J Am Coll Cardiol. 2019;73:1383-1385.
6. Geovanini GR, Libby P. Atherosclerosis and inflammation: overview and updates. Clin Sci (Lond). 2018;132:1243-1252.
7. Christia P, Frangogiannis NG. Targeting inflammatory pathways in myocardial infarction. European journal of clinical investigation. 2013;43:986-995.
8. Horckmans M, Ring L, Duchene J, Santovito D, Schloss MJ, Drechsler M, Weber C, Soehnlein O, Steffens S. Neutrophils orchestrate post-myocardial infarction healing by polarizing macrophages towards a reparative phenotype. Eur Heart J. 2017;38:187-197.
9. Peet C, Ivetic A, Bromage DI, Shah AM. Cardiac monocytes and macrophages after myocardial infarction. Cardiovasc Res. 2020;116:1101-1112.
10. Quillard T, Croce K, Jaffer FA, Weissleder R, Libby P. Molecular imaging of macrophage protease activity in cardiovascular inflammation in vivo. Thromb Haemost. 2011;105:828-836.
11. Mach F, Baigent C, Catapano AL, Koskinas KC, Casula M, Badimon L, Chapman MJ, De Backer GG, Delgado V, Ference BA, Graham IM, Halliday A, Landmesser U, Mihaylova B, Pedersen TR, Riccardi G, Richter DJ, Sabatine MS, Taskinen MR, Tokgozoglu L, Wikiund O, Group ESCSD. 2019 ESC/EAS Guidelines for the management of dyslipidaemias: lipid modification to reduce cardiovascular risk. Eur Heart J. 2020;41:111-188.
12. Williams B, Mancia G, Spiering W, Agabiti Rosei E, Azizi M, Burnier M, Clement DL, Coca A, de Simone G, Dominiczak A, Kahan T, Mahfoud F, Redon J, Ruilope L, Zanchetti A, Kerins M, Kjeldsen SE, Kreutz R, Laurent S, Lip GYH, McManus R, Narkiewicz K, Ruschitzka F, Schmieder RE, Shlyakhto E, Tsioufis C, Aboyans V, Desormais I, Group ESCSD. 2018 ESC/ESH Guidelines for the management of arterial hypertension. Eur Heart J. 2018;39:3021-3104.
13. Marx N, Federici M, Schutt K, Muller-Wieland D, Ajjan RA, Antunes MJ, Christodorescu RM, Crawford C, Di Angelantonio E, Eliasson B, Espinola-Klein C, Fauchier L, Halle M, Herrington WG, Kautzky-Willer A, Lambrinou E, Lesiak M, Lettino M, McGuire DK, Mullens W, Rocca B, Sattar N, Group ESCSD. 2023 ESC Guidelines for the management of cardiovascular disease in patients with diabetes. Eur Heart J. 2023
14. Visseren FLJ, Mach F, Smulders YM, Carballo D, Koskinas KC, Back M, Benetos A, Biffi A, Boavida JM, Capodanno D, Cosyns B, Crawford C, Davos CH, Desormais I, Di Angelantonio E, Franco OH, Halvorsen S, Hobbs FDR, Hollander M, Jankowska EA, Michal M, Sacco S, Sattar N, Tokgozoglu L, Tonstad S, Tsioufis KP, van Dis I, van Gelder IC, Wanner C, Williams B, Societies ESCNC, Group ESCSD. 2021 ESC Guidelines on cardiovascular disease prevention in clinical practice. Eur Heart J. 2021;42:3227-3337.
15. Knuuti J, Wijns W, Saraste A, Capodanno D, Barbato E, Funck-Brentano C, Prescott E, Storey RF, Deaton C, Cuisset T, Agewall S, Dickstein K, Edvardsen T, Escaned J, Gersh BJ, Svitil P, Gilard M, Hasdai D, Hatala R, Mahfoud F, Masip J, Muneretto C, Valgimigli M, Achenbach S, Bax JJ, Group ESCSD. 2019 ESC Guidelines for the diagnosis and management of chronic coronary syndromes. Eur Heart J. 2020;41:407-477.
16. Byrne RA, Rossello X, Coughlan JJ, Barbato E, Berry C, Chieffo A, Claeys MJ, Dan GA, Dweck MR, Galbraith M, Gilard M, Hinterbuchner L, Jankowska EA, Juni P, Kimura T, Kunadian V, Leosdottir M, Lorusso R, Pedretti RFE, Rigopoulos AG, Rubini Gimenez M, Thiele H, Vranckx P, Wassmann S, Wenger NK, Ibanez B, Group ESCSD. 2023 ESC Guidelines for the management of acute coronary syndromes. Eur Heart J. 2023
17. McDonagh TA, Metra M, Adamo M, Gardner RS, Baumbach A, Bohm M, Burri H, Butler J, Celutkiene J, Chioncel O, Cleland JGF, Coats AJS, Crespo-Leiro MG, Farmakis D, Gilard M, Heymans S, Hoes AW, Jaarsma T, Jankowska EA, Lainscak M, Lam CSP, Lyon AR, McMurray JJV, Mebazaa A, Mindham R, Muneretto C, Francesco Piepoli M, Price S, Rosano GMC, Ruschitzka F, Kathrine Skibelund A, Group ESCSD. 2021 ESC Guidelines for the diagnosis and treatment of acute and chronic heart failure. Eur Heart J. 2021;42:3599-3726.
18. McDonagh TA, Metra M, Adamo M, Gardner RS, Baumbach A, Bohm M, Burri H, Butler J, Celutkiene J, Chioncel O, Cleland JGF, Crespo-Leiro MG, Farmakis D, Gilard M, Heymans S, Hoes AW, Jaarsma T, Jankowska EA, Lainscak M, Lam CSP, Lyon AR, McMurray JJV, Mebazaa A, Mindham R, Muneretto C, Francesco Piepoli M, Price S, Rosano GMC, Ruschitzka F, Skibelund AK, Group ESCSD. 2023 Focused Update of the 2021 ESC Guidelines for the diagnosis and treatment of acute and chronic heart failure. Eur Heart J. 2023
19. Frangogiannis NG. Pathophysiology of Myocardial Infarction. Compr Physiol, 2015;5:1841- 1875.
20. Libby P, Tabas I, Fredman G, Fisher EA. Inflammation and its resolution as determinants of acute coronary syndromes. Circ Res. 2014;114:1867-1879.
21. Shinagawa H, Frantz S. Cellular immunity and cardiac remodeling after myocardial infarction: role of neutrophils, monocytes, and macrophages. Curr Heart Fail Rep. 2015;12:247-254.
22. Delaunay M, Osman H, Kaiser S, Diviani D. The Role of Cyclic AMP Signaling in Cardiac Fibrosis. Cells. 2019;9
23. Raker VK, Becker C, Steinbrink K. The cAMP Pathway as Therapeutic Target in Autoimmune and Inflammatory Diseases. Frontiers in immunology. 2016;7:123*.*
24. Bohn T, Rapp S, Luther N, Klein M, Bruehl TJ, Kojima N, Aranda Lopez P, Hahlbrock J, Muth S, Endo S, Pektor S, Brand A, Renner K, Popp V, Gerlach K, Vogel D, Lueckel C, Arnold-Schild D, Pouyssegur J, Kreutz M, Huber M, Koenig J, Weigmann B, Probst HC, von Stebut E, Becker C, Schild H, Schmitt E, Bopp T. Tumor immunoevasion via acidosis-dependent induction of regulatory tumor-associated macrophages. Nat Immunol. 2018;19:1319-1329.
25. Andreadou I, Cabrera-Fuentes HA, Devaux Y, Frangogiannis NG, Frantz S, Guzik T, Liehn E, da CP, Gomes C, Schulz R, Hausenloy DJ. Immune cells as targets for cardioprotection: New players and novel therapeutic opportunities. Cardiovasc Res. 2019
26. Frangogiannis NG. The inflammatory response in myocardial injury, repair, and remodeling. Nat Rev Cardiol. 2014;11:255-265.
27. Wen H, Peng L, Chen Y. The effect of immune cell-derived exosomes in the cardiac tissue repair after myocardial infarction: Molecular mechanisms and pre-clinical evidence. J Cell Mol Med. 2021;25:6500-6510.
28. Göbel et al., Rationale, design and baseline characteristics of the MyoVasc study: A prospective cohort study investigating development and progression of heart failure Eur J Prev Cardiol. 2021;28(9):1009-1018.

## Claims

1. An inductor of G-protein coupled receptor (GPCR)-mediated acidic stress response in cardiac or circulating bone marrow-derived monocytes or macrophages, wherein the inductor mediates induction of inducible cAMP early repressor (ICER), GPCR65 and/or GPCR132 for use in the prevention or treatment of heart failure (HF).

2. The inductor for the use according to claim 1, wherein the heart failure is selected from the group consisting of ischemic HF (IHF), myocardial infarction (MI), IHF resulting from acute and/or ongoing MI, HF with reduced ejection fraction (HFrEF), or HF with preserved ejection fraction (HFpEF), HF resulting from vascular dysfunction, disturbed tissue homeostasis and/or tissue acidification.

3. The inductor for the use according to claim 1 or claim 2, wherein the inductor is an expression construct, a proximity inducing drug, CREM, CD68, CSF1R or HIF-1α promoter, RNA stabilizer, transcription factor, or an enhancer of ICER, GPCR65 or GPCR132 gene or protein expression or agonist of GPCR65 or GPCR132.

4. The inductor for the use according to claim 3, wherein the enhancer for ICER is selected from the group consisting of cAMP response element (CRE) enhancers, NK-κb enhancers, HIF-1α enhancers or CREB-dependent calcium-responsive element enhancers.

5. The inductor for the use according to claim 3, wherein the enhancer for GPCR65 is selected from the group consisting of HIF-1α enhancers, hypoxia-inducible elements (HRE) enhancers, NF-κB and AP-1 enhancers, or PU.1 and IRF8-associated enhancers.

6. The inductor for the use according to claim 3, wherein the enhancer for GPCR132 is selected from the group consisting of PPARγ-responsive enhancers, HIF-1α enhancers, hypoxia-inducible elements (HRE) enhancers, or NF-κB and STAT3 enhancers.

7. The inductor for the use according to claim 1, wherein the inductor is inducible by an acidic pH environment.

8. The inductor for the use according to claim 1, wherein the inductor is a mRNA or cDNA encoding ICER, GPCR65 or GPCR132.

9. The inductor for the use according to claim 8, wherein said mRNA is delivered to cardiac or circulating bone marrow-derived monocytes or macrophages by means of lipid nanoparticles (LNPs), polymeric nanoparticles, cationic peptides, exosomes or extracellular vesicles (EVs), functionalized gold nanoparticles, electroporation or hydrogel-based delivery.

10. The inductor for the use according to claim 8, wherein said cDNA is delivered to cardiac or circulating bone marrow-derived monocytes or macrophages by means of a viral delivery vehicle selected from the group consisting of adeno-associated viral vector (AAV), adenoviral vector, helper-dependent adenoviral vector (HdAd), lentiviral vector or Sendai virus vector.

11. A vehicle for delivering a target mRNA or cDNA to cardiac or circulating bone marrow-derived monocytes or macrophages, wherein said target mRNA or cDNA encodes an inductor of G-protein coupled receptor (GPCR)-mediated acidic stress response in cardiac or circulating bone marrow-derived monocytes or macrophages, wherein said inductor modulates expression of inducible cAMP early repressor (ICER), GPCR65 and/or GPCR132 in said cardiac or circulating bone marrow-derived monocytes or macrophages.

12. A vehicle for delivering a target mRNA or cDNA to cardiac or circulating bone marrow-derived monocytes or macrophages according to claim 11 for use in the prevention or treatment of heart failure.

13. The vehicle for the use according to claim 12, wherein the inductor is anyone as defined in claims 1 to 10.

14. The vehicle for the use according to claim 12, wherein the vehicle is
a. a non-viral delivery vehicle selected from the group consisting of lipid nanoparticles (LNPs), polymeric nanoparticles, cationic peptides, monocyte or macrophage-derived exosomes or extracellular vesicles (EVs), functionalized gold nanoparticles, or
b. a viral delivery vehicle selected from the group consisting of adeno-associated viral vector (AAV), adenoviral vector, helper-dependent adenoviral vector (HdAd), lentiviral vector or Sendai virus vector.

15. The vehicle for the use according to any one of claims 12 to 14, wherein the HF is selected from the group consisting of IHF, MI, IHF resulting from acute and/or ongoing MI, HFrEF, or HFpEF, HF resulting from vascular dysfunction, disturbed tissue homeostasis and tissue acidification.

16. A pharmaceutical composition comprising an inductor as defined in anyone of claims 1 to 10, or a vehicle as defined in anyone of claims 12 to 15.

17. A monocyte or macrophage cell, comprising a gene encoding ICER, GPCR65 or GPCR132, wherein said cell is transfected with a vehicle comprising an inductor of ICER, GPCR65 or GPCR132 gene or protein expression as defined in any one of claims 1 to 10.
